Europäisches Patentamt

(19) European Patent Office

Office européen des brevets

(11) Publication number: **0 255 000**

**A2**

(12) **EUROPEAN PATENT APPLICATION**

(21) Application number: **87110377.6**

(22) Date of filing: **17.07.87**

(51) Int. Cl.⁴: **C01B 33/16 , A61K 7/16**

(30) Priority: **22.07.86 US 888899**

(43) Date of publication of application:
**03.02.88 Bulletin 88/05**

(84) Designated Contracting States:
**AT BE CH DE ES FR GB GR IT LI LU NL SE**

(71) Applicant: **W.R. GRACE & CO.**
**Grace Plaza 1114 Avenue of the Americas**
**New York New York 10036(US)**

(72) Inventor: **Rekas, Edward Paul**
**4245 Scarlet Sage Court**
**Ellicott City Maryland 21043(US)**
Inventor: **Harville, Cathy Linda**
**1678 Preakness Drive**
**Gambrills Maryland 21054(US)**
Inventor: **Stokes, Cary Michael**
**61 Chapeltowne Circle**
**Baltimore Maryland 21236(US)**
Inventor: **Cherigo, Charles Andrew**
**4255 Darleigh Road**
**Baltimore Maryland 21236(US)**

(74) Representative: **UEXKÜLL & STOLBERG**
**Patentanwälte**
**Beselerstrasse 4**
**D-2000 Hamburg 52(DE)**

(54) Silica aerogels and dentifrice compositions.

(57) Silica aerogels are prepared that have both abrasive and thickening properties when used in the preparation of dentifrice compositions.

EP 0 255 000 A2

## SILICA AEROGELS AND DENTIFRICE COMPOSITIONS

This invention relates to silica aerogels, and more particularly to silica aerogel compositions that may be used as abrasive and thickening agents.

Particulate silica compositions, including silica hydrogels, hydrous gels, aerogels and xerogels, have been used in the preparation of polishing and cleaning compositions for many years. In particular dentifrice compositions may contain a significant amount of an abrasive silica xerogel or hydrous gel to obtain the desired polishing qualities, and in addition a silica aerogel which serves to increase the viscosity (thicken) the composition to an acceptable level.

More recently, silica products have been developed which possess both thickening and abrasive properties. Such dual function products are desirable to the dentifrice industry, both from the standpoint of economy and ease of formulation.

U.S. 4,153,680 and 4,303,641 disclose hydrous silica gel compositions which are used in the preparation of dentifrice formulations. The hydrous gels are prepared by drying a silica hydrogel to a level of about 15-35% by weight and 20-60% by weight respectively.

G.B. 2,152,372 discloses dentifrices which contain a particulate dialytic silica abrasive/thickening agent which has a pore volume of about 0.1 to 6 cc/g. The dialytic silica is prepared by the electrodialysis of sodium silicate solution in the presence of silica containing nucleation seed particles.

While the prior art discloses the use of particulate silica products which will provide abrasive and or thickening properties, these products frequently are expensive or lack the ability to provide an acceptable level of both thickening and abrasive properties when used in reasonable amounts.

It is therefore an object of the present invention to provide particulate silica aerogel compositions which possess both abrasive and thickening properties.

It is another object to provide a method for obtaining particulate silica aerogels which may be used as an abrasive and/or a thickener in a wide variety of applications and products.

It is still further another object to provide dentifrice compositions which contain a silica aerogel that economically provides both polishing and viscosity modifying properties.

These and still further objects of the present invention will become readily apparent to one skilled in the art from the following detailed description and specific examples.

Broadly, our invention contemplates a novel silica aerogel which is characterized by both a significant degree of abrasiveness as well as thickening capability, particularly when used in the preparation of dentifrice compositions.

More specifically, we have discovered a novel silica aerogel product having the following physical characteristics:

(1) An internal (non-shrunk) nitrogen pore volume ($N_2$ PV) as measured by BET of 0.4 to 1.4 cc/g, and more preferably from about 0.4 to 1.0 cc/g.

(2) A surface area (SA), as measured by BET of 400 to 1000 $m^2/g$, and more preferably of 500 to 1000 $m^2/g$.

(3) A total volatiles content (TV), as measured gravimetrically of about 1 to 30 percent by weight, at 1750° for 1 hour and more preferably from 5 to 20 percent by weight.

(4) A Radioactive Dentine Abrasive value (RDA), as measured in accordance with the procedure of the American Dental Association described by Hefferson, J. Dent. Res. pp. 563-573 (1976) using 15 to 20% by weight silica in the paste and an RDA value of 100 for the calcium pyrophosphate reference standard, of 20 to 120, and more preferably 40 to 100.

(5) An average particle size range (APS), as measured by Microtrac, of 2 to 25 microns, and more preferably 5 to 15 microns.

(6) A density (D) of about 0.20 to 0.35 g/cc, and more preferably from 0.20 to 0.30 g/cc.

(7) An Oil Absorbtion of 90 to 180 and preferably 100 to 150 g/100 g of oil (linseed) as determined by ASTM D-281-31.

The novel silica aerogels of our invention are particularly useful for the preparation of dentifrice compositions. Typically, from about 5 to 50 percent by weight, and preferably 5 to 30 percent by weight of our novel silica aerogels may be combined with conventional dentifrice components such as humectants, surface active agents, flavoring agents and other silica products, preservatives, and coloring agents, as well as therapeutic agents such as flouride, antibiotics, germicides, and astringents to obtain commercially acceptable dentifrice compositions. U.S. patents 4,153,680 and 4,303,641 describe many conventional toothpaste components which may be included in the dentifrice composition contemplated herein.

The novel silica aerogels of the present invention are prepared as follows:

(I) Silica hydrogel is prepared by reacting an aqueous alkali metal silicate solution, preferably sodium silicate, with an aqueous inorganic acid, preferably sulfuric acid, in amounts to provide a stoichiometric excess of acid to obtain a reaction mixture pH of about 3 to 5, at a temperature of about 20 to 50°C for a period of 5 to 45 minutes.

(2) The resulting silica hydrogel which contains about 60 to 80 percent by weight water, is cut into pieces ranging from about 0.I0 to 20 $cm^3$ and washed with a solution of water for a period of I2 to 30 hours at a pH of 3.0 to 8.0, and a temperature of from about 60-88°C to obtain a washed hydrogel having a soda ($Na_2O$) content of about 0.0I to 0.5 weight percent.

(3) The washed hydrogel is then rapidly dried in a current of heated moving gas (air) at a temperature of 370 to 540°C for a period of 0.3 to 3 seconds which will provide a drying rate of from about 0.I to 2.3 g of water removed per g of hydrogel per second to obtain an aerogel which has a TV of from about I0 to 30 weight percent water and a retained original pore volume of from about 0.4 to I.4 cc/g.

(4) The resulting dried aerogel product which has been dried under the aforementioned rapid drying conditions which prevents full shrinkage of the gel structure, is then ground to a desired particle size of about 2 to 25 micrometer.

While the drying and grinding steps outlined above may be conducted separately, it is also contemplated that the washed hydrogel may be dried and ground simultaneously by using drying/milling devices such as for example, the Aljet Drier, Air Classifying Mill or Fluid Energy Mill; a drying/milling device where heated air conveys the gel as it is being milled. Furthermore, it is noted that sodium hydroxide solution may be added to the gel during drying and/or milling to increase the pH of the product.

The novel silica aerogel product of our invention is particularly distinguished by its ability to provide abrasive characteristics as well as thickening properties when used in the preparation of dentifrice compositions. It is our understanding that the aerogels of our invention possess these characteristics due to the fact the initial hydrogel structure is only partially shrunken during the rapid drying step. Therefore, the resulting products contain a significant degree of the initial (original) pore volume of the hydrogel which contributes to the desired thickening/abrasive properties of the product. In other words, since the hydrogel is only partially shrunken, the resulting aerogel product possesses desired abrasive properties which are frequently associated with a fully shrunken hydrogel (xerogel) product as well as surface area/thickening properties possessed by an aerogel.

Having described the basic aspects of the present invention, the following examples are given to illustrate specific embodiments thereof.

EXAMPLE I - Hydrogel Preparation

The silica hydrogel was prepared in the following manner: Aqueous solutions of sodium silicate (26 wt.% $SiO_2$ basis $SiO_2/Na_2O$ ratio 3.25 by weight) and sulfuric acid (43 wt.% $H_2SO_4$) were mixed at a ratio of 55 liters of silicate solution to I9 liters of sulfuric acid solution under I00 lbs. of pressure in a mixing nozzle. The gel mix was discharged onto a set belt, where the heat of reaction caused the gel temperature to peak at 46°C. The hydrogel remained on the belt for 35 minutes. It was then cut into pieces ranging from 0.I0 to 20 $cm^3$ and dropped into a wash tank.

EXAMPLE 2 - Wash Procedure

The hydrogel prepared in Example I was washed using the following procedures:

2A. Wash water solution was prepared by mixing sulfuric acid into water until pH = 5.5. The solution was heated to I40°F (60°C) and fed into a tank containing 4I00 kg of hydrogel at a rate of I32 liters/min. The washing was continued for 20 hours, at which time the tank was drained for 4 hours before discharging. The wash solution was discharged at a rate identical to the feed rate, and was recycled as many as three times.

2B. Wash water solution was prepared by mixing sulfuric acid into water until pH = 5.5. The solution was heated to 7I°C (I60°F). The water solution was fed into a tank containing 4I00 kg of hydrogel at a rate of I32 liter/min. The washing continued for 20 hours, at which time the tank was drained before discharging.

2C. Wash water solution was prepared by mixing sulfuric acid into water until pH = 3.2. The solution was heated to 82°C (I80°F) and fed into a tank containing 4I00 kg of hydrogel at a rate of I32 liters/min., with an equivalent wash water discharge rate. The solution was recycled as many as three times. The washing continued for I6 hours, at which time the tank was drained before discharging.

EXAMPLE 3 - Drying-Milling

The washed hydrogel of Example 2 was dried and milled simultaneously in a flash dryer using an inlet gas temperature of 400°C. The hydrogel feed rate was 1800 kg per hour. yielding 600 kg of 10 TV (wt.% total volatiles) finished product per hour. It was calculated that 0.33 g of water was removed per g of hydrogel per second. During the drying milling, the resulting aerogel was ground to an average particle size of 8-10 microns. Similar samples of washed hydrogel were oven dried at 250°F for 12 hours to obtain corresponding xerogel comparison samples.

EXAMPLE 4 - Properties

The data contained in the Table compares the aerogels of the present invention with xerogels which were prepared in a similar manner except that an equivalent quantity of water was removed from the hydrogel by slow oven drying. Caustic addition to the products during drying was used to achieve essentially neutral pH. In all cases, the xerogels have lower pore volumes, oil adsorptions, viscosities, and higher RDAs than the aerogels of the present invention, indicating that xerogels indeed have a significantly shrunken structure. The aerogels of the present invention, when added in amounts of 18 wt.% to a conventional toothpaste formulation (40% sorbitol, 25% glycerin, 6% Syloid 244 silica aerogel thickener, and the balance water, surfactant, flavor, preservative, color, etc.) provide significant higher thickening power to the dentifrice due to their higher pore volume compared to the xerogels.

TABLE

| | Aerogel A | Xerogel A | Aerogel B | Xerogel B | Aerogel C | Xerogel C |
|---|---|---|---|---|---|---|
| Wash Procedure, Example 2 | | | | | | |
| Drying Procedure, Example 3 | | | | | | |
| % Total Volatiles | 12.3 | 7.2 | 11.5 | 9.0 | 11.7 | 12.5 |
| pH | 7.3 | 7.6 | 7.0 | 6.6 | 6.7 | 6.4 |
| % $Na_2O$ | 0.16 | 0.13 | 0.17 | 0.10 | 0.15 | 0.16 |
| % $SO_4$ | 0.02 | 0.01 | 0.06 | 0.02 | 0.04 | 0.08 |
| Surface Area, $M^2/g$ | 765 | 593 | 725 | 648 | 823 | 657 |
| Pore Vol. – $N_2$, cc/g | 0.91 | 0.41 | 0.46 | 0.41 | 0.69 | 0.39 |
| Oil Ads, g/100 g | 181 | 121 | 121 | 79 | 144 | 102 |
| Avg. Particle Size, u | 9.5 | 8.1 | 7.6 | 8.7 | 9.0 | 10.2 |
| Toothpaste Viscosity Reading (Brookfield) | | | | | | |
| RVT @ rpm, E spindle | 85 | 27 | 33 | 17 | 38 | 18 |
| Paste RDA | 59 | 95 | 70 | 118 | 45 | 101 |

The above examples clearly indicate that the novel silica aerogel products of our invention may be used to prepare useful dentifrice compositions.

5

## Claims

1. A silica aerogel having a pore volume of from about 0.4 to 1.4 cc.g, a surface area of from about 400 to 1000 m²/g, and an RDA value of from about 20 to 120.

2. The silica aerogel of claim 1 having a water content of about 1 to 30 percent by weight.

3. The silica aerogel of claim 1 having an average particle size of from about 2 to 25 micrometers.

4. The silica aerogel of claim 1 having a density of from about 0.2 to 0.35 g·cc.

5. A dentifrice composition comprising from about 5 to 50 weight percent of silica aerogel of claim 1. and dentifrice components selected from the group consisting of humectants, theraputic agents, surfactants. flavoring agents and preservatives, and mixtures thereof.

6. In a method for preparing a silica aerogel wherein a hydrogel is washed. dried and milled. the improvement comprising conducting said washing step at a pH of 3 to 8 at a period of 12 to 30 hours at a temperature of 60-88°C, and rapidly drying the washed hydrogel at a temperature of 370 to 540°C to a moisture content of from about 10 to 30 weight percent.

7. The method of claim 6 wherein said drying step is conducted under conditions wherein water is removed from said gel at a rate of from about 0.1 to 2.3 g of water per g of gel per minute.

8. The method of claim 7 wherein water is removed from the internal structure of said gel to obtain a retained pore volume of about 0.4 to 1.4 cc/g.